# EUROPEAN PATENT SPECIFICATION

(11) **EP 2 141 145 B1**
(45) Date of publication and mention of the grant of the patent: **20.02.2013**
(21) Application number: 08011786.4
(22) Date of filing: 30.06.2008
(51) Int. Cl.: C07C 67/303, B01J 14/00

(54) **Efficient and highly enantioselective Rh-catalyzed hydrogenations of unsaturated lactate precursors with chiral bisphospholanes as ligands**
Effiziente und hoch-enantioselektive Rh-katalysierte Hydrierungen von ungesättigten Laktatvorläufern mit chiralen Bisphospholanen als Liganden
Hydrogénations efficaces et fortement énantiosélectives de précurseurs insaturés de lactate catalysées par Rh doté de bisphospholanes chiraux en tant que ligands

(43) Date of publication of application: 06.01.2010
(73) Proprietor: ThyssenKrupp Uhde GmbH, 44141 Dortmund (DE)
(72) Inventor: Schäffner, Benjamin, Dr., Palo Alto, CA 94306-3474 (US); Holz, Jens, Dr., 18196 Kessin (DE); Börner, Armin, Prof. Dr., 18059 Rostock (DE)

(56) References cited:
- WO-A-2007/051724
- GB-A- 1 592 536
- W.G. FISCHER: "Spaltrohr-Kolonnen zur hochwirksamen destillativen Trennung im Laboratorium" CHEMIE FÜR LABOR UND BETRIEB, vol. 27, 1976, pages 42-46, XP009110792
- BURK M J: "C2-SYMMETRIC BIS (PHOSPHOLANES) AND THEIR USE IN HIGLY ENANTIOSELECTIVE HYDROGENATION REACTIONS" JOURNAL OF THE AMERICAN CHEMICAL SOCIETY, AMERICAN CHEMICAL SOCIETY, WASHINGTON, DC.; US, US, vol. 113, 1 January 1991 (1991-01-01), page 8518/8519, XP002065437 ISSN: 0002-7863
- J. BAYARDON ET AL: "Propylene carbonate as a solvent for asymmetric hydrogenations" ANGEW.CHEM.INT.ED., vol. 46, 3 July 2007 (2007-07-03), pages 5971-5974, XP002511066
- KOENIG K E ET AL: "ASYMMETRIC HYDROGENATION OF GEMINAL-SUBSTITUTED VINYL ACETATES" JOURNAL OF ORGANIC CHEMISTRY, AMERICAN CHEMICAL SOCIETY, EASTON.; US, vol. 45, no. 12, 1 January 1980 (1980-01-01), pages 2362-2365, XP002933995 ISSN: 0022-3263
- BURK M J ET AL: "Rh-DuPHOS-Catalyzed Enantioselective Hydrogenation of Enol Esters. Application to the Synthesis of Highly Enantioenriched alpha-Hydroxy Esters and 1,2-Diols" JOURNAL OF THE AMERICAN CHEMICAL SOCIETY, AMERICAN CHEMICAL SOCIETY, WASHINGTON, DC.; US, US, vol. 120, no. 18, 1 January 1998 (1998-01-01), pages 4345-4353, XP002315073 ISSN: 0002-7863
- HOLZ JENS ET AL: "Synthesis of chiral 2,5-bis(oxymethyl)-functionalized bis(phospholanes) and their application in Rh- and Ru-catalyzed enantioselective hydrogenations" EUROPEAN JOURNAL OF ORGANIC CHEMISTRY, WILEY-VCH VERLAG, WEINHEIM, DE, 1 January 2001 (2001-01-01), pages 4615-4624, XP002303790 ISSN: 1434-193X

## Description

The invention relates to a process for asymmetric hydrogenation of α-acetoxy acrylates to enantiopure O-acetyl lactates with Rh-catalysts based on chiral bisphospholane ligands in propylene carbonate (PC) as green solvent and to the apparatus suitable for operating that process. In particular the invention integrates a Spaltrohrkolonne into that enantiopure O-acetyl lactate production process.

Enantiopure lactic acid plays an increasing role as building block for the synthesis of biodegradable chiral poly-(lactic acids) (PLA) which have a similar application range like polyethylene terephthalate (PET). Lactic acid is a sophisticated intermediate of the production of cleaning agents, liquid soaps and softening agents. Recently, the interest in enantiopure lactic acid was growing constantly. This is because of the biodegradability of the polymeric form PLA. PLA is generally used in biomedical applications, in tissue engineering and for the preparation of bio plastics,

In general the lactic acid monomer is produced from sugar feedstocks by fermentation. This includes the difficulty of extracting the generated lactic acid from the fermentation solution. In addition most of the known solvents are toxic to micro organisms, are uneconomic or possess inappropriate solvent features.

An alternative methodology to produce chiral enantiopure compounds is the chemical synthesis by asymmetric hydrogenation using homogeneous catalysis. This environmentally friendly technology has reached a high level of application in industry. Attainable high rates and selectivities has given rise to the use in chemical manufacturing of pharmaceuticals, animal health products, agrochemicals, flavours, pheromones, fungicides, and fragrances.

For this reaction a metal catalyst is required to decrease reaction temperature as well as pressure. Homogenous catalysts are dissolvable in the substrate containing solvent. In this regard homogenous rhodium(I), ruthenium(II) or iridium(I) catalysts based on chiral phosphorus ligands plays important roles. Lots of chiral ligands have been synthesized in the past to overcome limitations in the hydrogenation of standard. In general, chiral ligand can be classified by their coordination abilities (mono- or be-dentate, P-P, P-N etc.), by their backbone nature and by their source of chirality (atropisomers, metallocene, chiral C-skeleton, stereogenic phosphorus etc.). Improvement of catalyst ligands is mainly achieved by modifications of steric features (like the bite angle α) as well as by alteration of the electronic surrounding at the metal centre. This leads to families of ligands comprising strongly related members. Examples represent commercially available bisphospholanes of the catASium M series and those of the DUPHOS family in both enantiomeric forms that allows the production of L- and D-lactic acid. Appropriate ligands out of these both classes show superior behaviour in the Rh-catalyzed hydrogenation of a broad range of substrates, but only small structural changes causes striking differences in the catalytic features. Also, Rh-precatalysts are generated in propylene carbonate (PC).

Additionally, to the choice of the appropriate ligand of the catalyst the selection of the solvent is obligatory. Commonly used solvents like methanol, tetrahydrofuran, dichloromethane and toluene differ strongly in use and limitations. Fluorinated alcohols and ionic liquids are characterized by high prices, toxicity or problems with purification. Another class of solvents of interest are organic carbonates, especially cyclic carbonates. These carbonates are characterized by a high boiling point and a low vapour pressure. They are inflammable and show only low viscosity.

Propylene carbonate (PC) is of particular interest, because of its low toxicity and easy combustibility. According to these features propylene carbonate can be considered to be an ecologically and economically benign solvent ("green solvent"). Moreover it can be synthesised environmentally friendly out of propylene oxide (the latter can be derived from propylene and H₂O₂ via HPPO technology) by reaction with supercritical CO₂ using transition metal catalysts. Propylene carbonate belongs to a class of aprotic highly dipolar solvents (AHD). Its physical properties are highly unique and are only met by those of acetonitrile that do not belong to the AHD group Propylene carbonate has been established in industry for pharmaceutical and medical applications and as solvent for extractions and coating processes. In academic research propylene carbonate has been used for the stabilization of Pd clusters in Heck-reaction and as solvent in Rh-catalyzed hydroformylation reactions in temperature depending multi-component solvent systems (TMS). Propylene carbonate was also employed for the asymmetric heterogeneous Ir-catalyzed hydrogenation of ketones. Recently, propylene carbonate was identified as a beneficial solvent in Pd-catalyzed asymmetric allylic alkylations, aminations and in Sonogashira reactions.

The high boiling point (242°C) characterizes propylene carbonate as a green solvent, but may increase the intricacy of product separation. For unpolar substrates satisfactory solutions are established. For example, the extraction with n-hexane and the use of a TMS system are conventionally used. More polar products like alkylation and amination products can be separated from propylene carbonate via column chromatography with an eluent mixture n-hexane : ethyl acetate higher than 6 : 1. The distillation of products out of propylene carbonate under vacuum is also problematic, because oxygen-oxygen interactions between the product and propylene carbonate cause propylene carbonate to be codistilled.

An opportunity to improve distillation is to use a Spaltrohrkolonne. This method is also described in the literature as Spaltrohrcolumn, slit tube column, or with its detailled description Fischer HMS 500. The advantage of this entity is a series connection of multiple distillation devices for thermal separation of substances. The effect of separation in comparison to conventional distillation is improved because of the steam counter flow that allows multiple contact of the steam with the liquid sample.

The article of W.G. Fischer: "Spaltrohr-Kolonnen zur hochwirksamen Trennung im Laboratorium", Chemie fur Labor und Betrieb, Vol. 27. 1976, pages 42-46, XP009110792 relates to the use of a Spaltrohrkolonne for efficient separation of compound mixtures.

In Burk M.J:: "C2 symmetric bis(phosophlanes) and their use in highly enantioselective hydropenation reactions". Journal of the American Chemical Society, American Chemical Society, Washington, DC, US, Vol. 113, 1 Januarv 1991, page 8518/8519, XP002065437 ISSN: 002-7863 relates to the asymmetric hydrogenation of a prochiral alpha-acetoxy acrylic acid using Rhodium catalysts with chiral phosphine ligands.

None of these documents describes the use of the combination of the Spaltrohrkolonne in connection with the use of propylene carbonate in the asymmetric hydrogenation of prochiral alpha-acetoxy acrylic acid.

The task of the invention therefore is 1) to identify propylene carbonate (PC) as a suitable solvent for the asymmetric hydrogenation of α-acetoxy acrylates to O-acetyl lactates, 2) to identify appropriate ligands for Rh-catalysts in propylene carbonate for the hydrogenation of α-acetoxy acrylates, and 3) to separate O-acetyl lactates from propylene carbonate by Spaltrohrkolonne. A further task of the invention is to make the process more environmentally friendly. And also a task of the present invention is to optimize the conversion into the end product resulting in high yields and in enantiopure stereoisomers without remaining propylene carbonate-impurities. Further tasks are to make the process cost-effective and to keep reaction times as short as possible but thereby implementing the latter terms and conditions. It is also object of this invention to provide the apparatus suitable to operate such a process.

This is achieved by a process for asymmetric hydrogenation of prochiral α-acetoxy acrylic acid esters for the synthesis of enantiopure lactic acid esters by the use of Rh-catalyzed bisphospholane ligands in a unit using an autoclave, and a distillation process. In a first process step α-acetoxy acrylic acid esters (1) and Rh-catalyzed bisphospholane ligands are dissolved in a solvent as substrate solution,
in a second process step the substrate solution is added to an evacuated autoclave, in a third process step H₂ is fed into the autoclave, in a fourth process step the asymmetric hydrogenation reaction is carried out under H₂-pressure, and in a fifth process step the reaction product composed of solvent, lactic acid esters (2), remaining α-acetoxy acrylic acid esters (1) and Rh-catalyzed bisphospholane ligands is transferred into a distillation unit, is being distilled and enantiopure lactic acid esters (2) are separated from solvent, α-acetoxy acrylic acid esters (1) and Rh-catalyzed bisphospholane ligands, In this process propylene carbonate is used as solvent in the first process step, and the distillation is performed in a Spaltrohrkolonne in the fifth process step.

Further embodiments of the invention relate to the types of heterocyclic phosphine ligands used in this process. These ligands can be chosen out of the DuPHOS family (5a, 5b) and the catAsium M series (10a, 10c, 10d), BASPHOS (8), or Ferrotane (12). Depending on the used enantiomeric form of the ligand of the catalyst pure D- or L-enantioselective O-acetyl lactates (2) can be generated.

Optionally, Rh-catalysts are synthesized as Rh(L)(1,5-cyclooctadiene)]BF₄ or [Rh(L)(norbomadiene)]BF₄ prior to use as described in WO 2005/049629 A1 or are used from chemical suppliers without further purification. (L) stands for heterocyclic phosphine ligands in which (L) stands for heterocyclic phosphine ligands that will be bound to the catalyst and then pronounced as Rh-catalyzed bisphospholane ligands.

A further option of the current invention in the first process step is that the Rh-catalyzed bisphospholane ligands are put into the autoclave in their solid state, before evacuation. This approach ensures that all traces of oxygen can be removed in the autoclave.

Further embodiments of the invention relate to the reaction conditions in the fourth process step. The asymmetric hydrogenation in the autoclave is being carried out at an initial H₂-pressure of 1 - 20 bar and a temperature of 20 - 40 °C. The substrate solution contains a concentration of 0.4 to 0.5 mmol/mL α-acetoxy acrylic acid esters (1) and 1 mol% Rh-catalyzed bisphospholane ligands. To obtain improved enantioselective yields the ratio of catalyst and -acetoxy acrylic acid esters (1) is important.

Optionally, the ethyl ester group of the prochiral substrate can be replaced by a methyl ester group. This substitution optimizes the atom economy and therefore the environmental friendliness of the reaction. Both, ethyl O-acetoxy acrylate and methyl O-acetoxy acrylate are synthesized as described in Fryzuk, M.D. et al., J. Am. Chem. Soc. 1978, 100, 5491 - 5494.

Further embodiments of to the invention are the distillation temperature and pressure settings of the fifth process step. These are defined as an oil bath temperature in the range of 107 - 112°C, a sump temperature in the range of 90 - 98 °C, a shell temperature of the Spaltrohrkolonne in the range of 70 - 90 °C, a distillation temperature at the top of the Spaltrohrkolonne of 52 - 95 °C, and the distillation pressure being selected from a pressure range of 6.5 to 9 mbar. A pressure less than 6 mbar is not recommended because under this condition the product will not be passed into liquid phase. The ideal distillation conditions for methyl-2-acetoxyacrylat are the following parameters: an oil bath temperature of 107 °C, a sump temperature of 94 °C, a temperature of the shell side of the Spaltrohrkolonne of 80 °C, a distillation temperature at the top of the Spaltrohrkolonne of 52.5 °C. The same ideal distillation conditions are true for ethyl-2-acetoxyacrylat but the distillation temperature at the top of the Spaltrohrkolonne is 58 °C. Therefore the optimal distillation range is tightened for ethyl-2-acetoxyacrylat resulting in fewer yields.

The described process of asymmetric hydrogenation with separation of the enantiopure lactic acid esters is to be operated in an apparatus comprising an autoclave, a Spaltrohrkolonne, a means for conveying the substrate solution into the autoclave, a means for evacuation of the autoclave, a means for transferring the reaction product to the Spaltrohrkolonne, a means for the collection of the separated end product, and a means for the collection of solvent, catalyst and remaining starting material.

Optionally, the apparatus is of isobaric and/or isothermic nature and has an automatic hydrogen detection unit. Both features allow easy controlling of the reaction process.

In the following, the invention is illustrated by 3 figures summarizing the underlying issues.

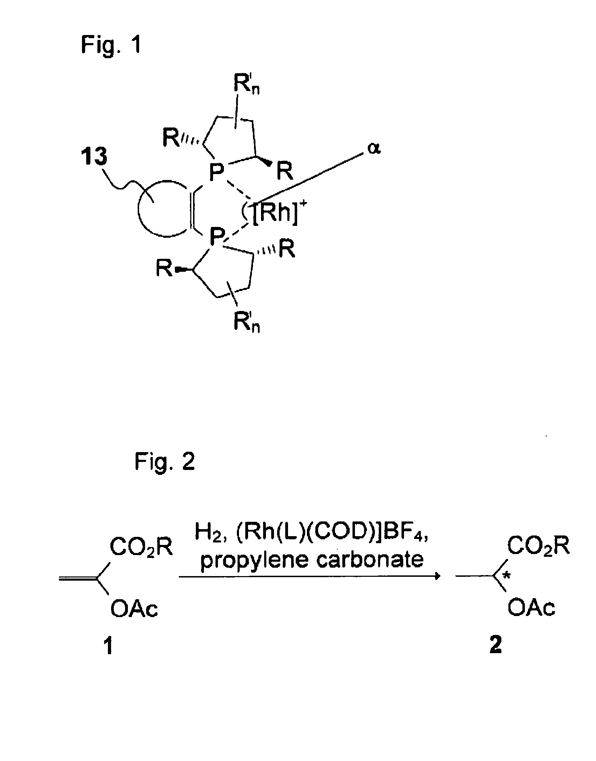

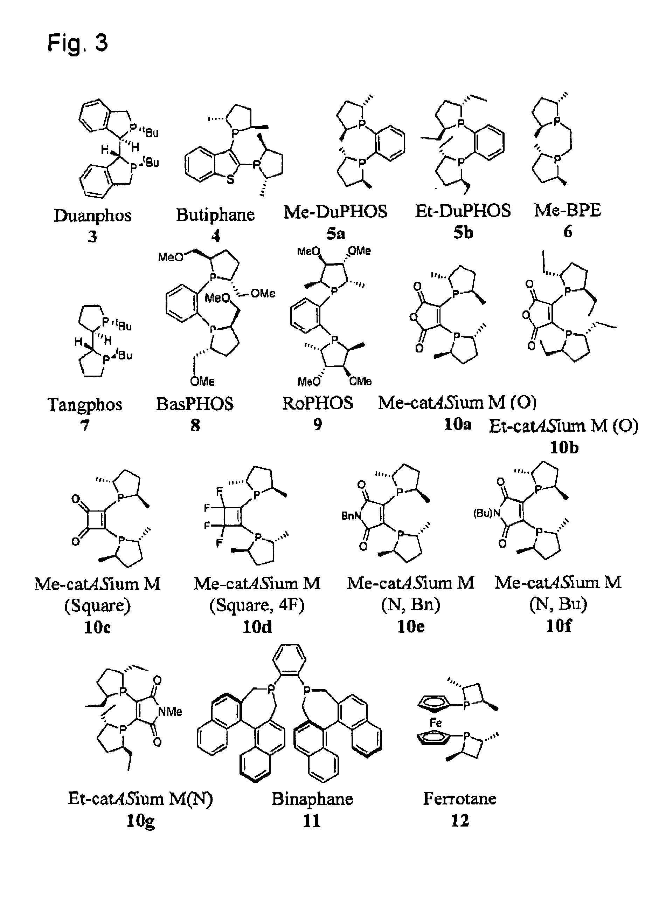

Fig. 1 shows the constitution of bisphospholane Rh-catalysts. In this structure R is selected from a group comprising an alkyl-group (C₁ - C₃), a phenyl group, or a CH₂OR"-group. In said CH₂OR"-group R" is an H-group, an alkyl-group (C₁ to C₅), or a benzyl-group. R'ₙ is referred to be an H-group, or an OR"'-group with n being 1 or 2.. In said OR"'-group R''' is an alkyl-group (C1-C3), or a benzyl-group. P stands for phosphorus. There are two different opportunities for electronic or steric fine tuning of the skeletal structure of such catalyst. First, the bite angle α can be modified and thus influence the steric features of the catalyst. Second, the electronic surrounding at the metal centre can be altered. Both modifications lead to striking differences in the catalytic features of these compounds. Alternatively to the shown cyclic structure (13) of the Rh-catalyst an acyclic structure can be introduced. Examples for cyclic groups are four- or five-membered ring structures as shown in ligands (10a), (10c) in Fig. 3, or aromatic structures as shown by way of example in ligand (5a) in Fig. 3. Examples for acyclic groups are ethylene or propylene bridges as shown in ligands (3), (6) or (7) in Fig. 3.

Fig. 2 shows the general hydrogenation of lactic acid precursors (1) by the use of Rh-catalyst in propylene carbonate (PC). Lactic acid precursors (1) are dissolved in propylene carbonate plus ligand bound catalyst. The reaction is carried out under an initial H₂ pressure of 1 - 10 bar. Hereby R is an ethyl ester group or a methyl ester group. (L) in [Rh(L)(COD)]BF₄ stands for different ligands that can be bound to the catalyst. These ligands will be selected out of the range presented in Fig. 3(3-12). (COD) in [Rh(L)(COD)]BF₄ stands for 1,5 cyclooctadiene but can be substituted by norbornadiene.

Fig. 3 shows the different heterocyclic phosphine ligands (3 - 12) considered in this invention. These ligands comprise many structural and electronic variations.

The invention is further exemplified by 5 tables summarizing results of the underlying reactions.

Table 1 shows the reaction of chiral phosphine ligands in the Rh-catalyzed hydrogenation of ethyl O-acetoxy acrylate (1).
10 bar initial H₂ pressure, 1 mol% [Rh(L)(1,5 Cyclooctadiene)]BF₄ catalyst, 0.4 mmol ethyl O-acetoxy acrylate in 2 mL propylene carbonate are used. Amount of conversion and amount of enantioselectivity are given in %. Ligand numbering corresponds to the numbering introduced in Fig. 3. (R) is referred to *(R)*-O-acetyl ethyl lactate, and (S) is referred to *(*S*)*-O-acetyl ethyl lactate.

| Ligand Nr. | Conversion [%] | enantioselectivity [%] |
|---|---|---|
| 3 | 7 | 75 (R) |
| 4 | 4 | 42 (S) |
| 5a | > 99 | 89 (S) |
| 5b | > 99 | > 99 (S) |
| 6 | 5 | 19 (R) |
| 7 | 4 | 42 (S) |
| 8 | > 99 | > 99 (S) |
| 9 | 2 | 35 (S) |
| 10a | > 99 | 97 (R) |
| 10e | 4 | 43 (R) |
| 10d | > 99 | 65 (S) |
| 10c | > 99 | 81 (R) |
| 10f | 2 | 21 (R) |
| 11 | > 99 | 95 (S) |
| 12 | 52 | 49 (S) |

It is shown that small changes in the structure of the bisphospholanes result in dramatic effects on the catalytic reaction. Rh-catalysts based on the ligands Me-DuPHOS (5a), Et-DuPHOS (5b), BOSPHOS (8), and Me-cat*A*Sium M (O) (10a) are very active. Binaphane (11) also shows good performance.

Table 2 shows the Rh-catalyzed hydrogenation of ethyl *O-*acetoxy acrylate (1) with Me-DuPHOS (5a) and Me-cat*A*Sium M(O) (10a) as ligands carried out at different initial pressure and varying solvents. 1 mol% [Rh(L)(1,5 Cyclooctadiene)]BF₄ catalyst, 0.4 mmol ethyl *O-*acetoxy acrylate (1) in 2 mL solvent are used. ²[Rh(cat*A*Sium M)(norbornadiene)]BF₄ is used; ³ catalyst is prehydrogenated for 1 h in propylene carbonate ; ⁴ The precatalyst was stirred for 24 h in MeOH before use; 5 GC = glycerine carbonate. Ligand numbering corresponds to the numbering introduced in Fig. 3. *(R)* is referred to *(R)*-O-acetyl ethyl lactate, and *(S)* is referred to *(S)*-O-acetyl ethyl lactate.

| Ligand Nr. | initial pressure H₂ [bar] | reaction time [min] | solvent | Conversion [%] | enantioselectivity [%] |
|---|---|---|---|---|---|
| 5a | 1 | 1175 | PC | > 99 | 90 (S) |
| 5a | 10 | 40 | PC | > 99 | 89 (S) |
| 5a | 1 | 240 | MeOH | > 99 | 97 (S) |
| 5a | 10 | 40 | MeOH | > 99 | 98 (S) |
| 5a | 1 | 795 | CH₂Cl₂ | 52 | 92 (S) |
| 5a | 10 | 40 | CH₂Cl₂ | 73 | 65 (S) |
| 5a | 1 | 780 | THF | > 99 | > 99 (S) |
| 5a | 10 | 40 | THF | 87 | 96 (S) |
| 10a | 1 | 80 | PC | > 99 | 98 (R) |
| 10a | 1 | 70² | PC | > 99 | 98 (R) |
| 10a | 1 | 70^{2,3} | PC | > 99 | 98 (R) |
| 10a | 10 | 40 | PC | > 99 | 97 (R) |
| 10a | 10 | 40² | PC | > 99 | 98 (R) |
| 10a | 1 | 325 | MeOH | > 99 | 88 (R) |
| 10a | 10 | 40 | MeOH | > 99 | 95 (R) |
| 10a | 10 | 40⁴ | MeOH | > 99 | 95 (R) |
| 10a | 1 | 50 | CH₂Cl₂ | 43 | 96 (R) |
| 10a | 10 | 40 | CH₂Cl₂ | > 99 | 94 (R) |
| 10a | 1 | 800 | THF | 35 | 94 (R) |
| 10a | 10 | 40 | THF | > 99 | 97 (R) |
| 10a | 1 | 210 | GC⁵ | > 99 | 92 (R) |

Both ligands, Me-DuPHOS (5a) and Me-catASium (O) (10a), show good to excellent enantioselectivites independent of which of the following solvent is used: propylene carbonate, methanol, methylene chloride, tetrahydrofuran or glycerine carbonate.

But reaction time depends clearly on the used Rh-ligand and initial H₂ pressure. At 10 bar initial hydrogen pressure in combination with Rh(Me-DuPHOS)-catalyst (5a) leads to fast reactions in all solvents, whereas hydrogenation is slowed down by a factor of 5 to 30 at 1 bar initial H₂-pressure. Enantioselectivity is highest by the use of methanol as solvent.

If Me-cat*A*Sium M (O) (10a) is used the pressure effect is much less pronounced. Also results with propylene carbonate as solvent are better then those done in methanol as solvent. Only a slight deceleration of the reaction in propylene carbonate at 1 bar in comparison to 10 bars can be observed. This behaviour is unique in comparison to other solvents. For example, in tetrahydrofuran or methylene chloride the reaction does not come to completion. The enantioselectivity is constantly high in all conditions in propylene carbonate.

The use of [Rh(cat*A*Sium M)(norbornadiene)]BF₄ as precatalyst further decreased reaction time. It is a well known effect, that reaction times with precatalysts bearing 1,5-cyclooctadiene as stabilizing counterligand are characterized by longer reaction times in comparison to catalysts characterized by a norbornadiene constitution.

Processes in presence of propylene carbonate are more efficient in comparison to the use of MeOH and H₂O as solvent, because the ring opening of the anhydride unit of the ligand can be avoided.
By way of example, such a reaction can be investigated by ³¹P-NMR measurements. ¹H and ¹³C NMR data are taken in deuterated chloroform and MeOH-d⁴ at 300 MHz. Proton chemical shifts are referenced to TMS standard. Carbon chemical shifts are referred to the carbon signal of the solvent at 77.0 ppm.

Table 3 shows the Rh-catalyzed hydrogenation of ethyl *O-*acetoxy acrylate (1) in propylene carbonate with Et-cat*A*Sium M (O) (10b) and Et-cat*A*Sium M (N) (10g) as ligands. 1 mol% [Rh(L)(1,5 Cyclooctadiene)]BF₄ catalyst, 0.4 mmol ethyl O-acetoxy acrylate (1) in 2 mL solvent are used. Ligand numbering corresponds to the numbering introduced in Fig. 3. *(R)* is referred to *(R)*-O-acetyl ethyl lactate, and *(S)* is referred to *(S)*-O-acetyl ethyl lactate.

| Ligand Nr. | initial pressure [bar] | reaction time [min] | Conversion [%] | enantioselectivity [%] |
|---|---|---|---|---|
| 10b | 1 | 70 | > 99 | > 99 (S) |
| 10b | 10 | 40 | > 99 | > 99 (S) |
| 10g | 1 | 210 | > 99 | > 99 (S) |

The use of Et-cat*A*Sium M (O) (10b) or Et-cat*A*Sium M (N) (10g) results in optimal enantioselectivites. Ligand 10b catalysis reaction approx. 3 fold faster than the ligand 10g. Therefore, the steric effect exerted by bulky alkyl groups in 2 and 5 positions of the phospholanes is pivotal for the achievement of high enantioselectivity.

Table 4 shows the Rh-catalyzed hydrogenation of methyl *O-*acetoxy acrylate (1) in propylene carbonate with Me-cat*A*Sium M ligands (10a, 10b), with ligand of the DuPHOS family (5a, 5b) as well as with BASPHOS (8). 1 mol% [Rh(L)(1,5 Cyclooctadiene)]BF₄ catalyst, 0.4 mmol methyl *O-*acetoxy acrylate in 2 mL solvent are used. The ligand BASPHOS (8) was used in combination with the precatalyst [Rh(L)(norbomadiene)]BF₄. Ligand numbering corresponds to the numbering introduced in Fig. 3. *(R)* is referred to *(R)*-O-acetyl methyl lactate, and (S) is referred to *(S)*-O-acetyl methyl lactate.

| Ligand Nr. | initial pressure [bar] | reaction time [min] | Conversion [%] | enantioselectivity [%] |
|---|---|---|---|---|
| 5a | 10 | 40 | 28 | 87 (S) |
| 5b | 10 | 40 | > 99 | 98 (S) |
| 8² | 10 | 40 | > 99 | 98 (S) |
| 10a | 10 | 40 | > 99 | 95 (R) |
| 10b | 10 | 40 | > 99 | 98 (S) |

Optimal asymmetric hydrogenation is still possible after replacement of the ethyl by a methyl ester group in the prochiral substrate (1) if the following ligands are used: Et-DuPHOS (5b), BASPHOS (8), Me-cat*A*Sium M (O) (10a), Et-cat*A*Sium M (O) (10b). But this is not true for Me-DuPHOS (5a) if used as catalyst ligand in combination with the prochiral substrate containing a methyl ester group.

In the processes defined above, in which hydrogenations at an initial pressure of 10 bar are described, an isothermic hydrogenation device with automatic hydrogen pressure detection is used. 1 mol% catalyst is filled in the autoclave and it is evacuated three times followed by hydrogen flushing. 0.4 mmol substrate is dissolved in 2 mL solvent and filled into the autoclave via a syringe.

In the processes defined above, in which hydrogenations at an initial pressure of 1 bar are described, an isothermic and isobaric hydrogenation device with automatic hydrogen consumption detection is used. 1 mol% catalyst is filled in the autoclave and it is evacuated three times followed by hydrogen flushing. 0.4 mmol substrate is dissolved in 2 mL solvent and filled into the autoclave via a syringe.

For determination of conversion yield and amount of enantioselectivity a chiral GC (50m Chiraldex β-pm; Astec) is used.
Conversion rates of 100 % can be achieved using Me- or Et-cat*A*Sium M-ligands. Enantioselectivites of 99 % can be achieved, if ethyl O-acetoxy acrylate (1) in propylene carbonate is used and the hydrogenation is performed in 1 to 10 bar initial H₂ pressure.

Table 5 shows the separation of ethyl *O-*acetoxy acrylate and methyl *O-*acetoxy acrylate from propylene carbonate by Spaltrohrkolonne.
The product mixture is distilled using a HMS 500 C Spaltrohrkolonne from Fischer technology. All experiments are accomplished with a mixture of 100 mL propylene carbonate with 6.7 g of ethyl *O-*acetoxy acrylate or methyl *O-*acetoxy acrylate (and mixtures) (1). [a] Yield in comparison to maximum compound which can be distilled; [b] Determined by 1 H-NMR (300 MHz, CDCl3); [c] The experiment is done with mixtures of 32 mL propylene carbonate and 812 mg product; The product fraction is collected in a cooled flask (-78°C); [d] A 100 mL solution, containing 6.7 g of a mixture. The previously done hydrogenation is stopped after 70 % conversion to analyze the separation of product and starting material in mixtures. The fraction is free of propylene carbonate but contains a mixture of starting material ; product = 14 : 86 et %. Ligand numbering corresponds to the numbering introduced in Figure 2.

| Entry | vacuum [mbar] | temperature bath [°C] | temperature solution [°C] | temperature isolation [°C] | yield [g] | [%]^{[a]} | purity^{[b]} [wt%] |
|---|---|---|---|---|---|---|---|
| 1 | 0.12 | 65 | 58 | 24 | 0.88^{[c]} | (96) | 89 |
| 2 | 7.5 | 107 | 94 | 53 | 5.78 | (86) | 100 |
| 3 | 7.5 | 107 | 94 | 55 | 5.31 | (79) | 100 (14:86)^{[d]} |
| 4 | 7.5 | 107 | 94 | 58 | 5.15 | (77) | 100 |

An improved separation can be achieved at 7.5 mbar vacuum. The end product is free of propylene carbonate impurities and 86 % can be distilled out of the reaction phase (entry 2). The pure end product is enriched in the first fraction while non-converted starting material Is found in the second fraction (entry 3).

Determination of purity of end product is achieved by ¹H-NMR at 300 MHz in deuterated chloroform.
The process described above leads to 77 % yield of ethyl O-acetoxy acrylate and 79 - 96 % yield of methyl O-acetoxy acrylate and thus leads to a successful separation of the end product from propylene carbonate.

A further example for the invention is given in the following work protocol: A 2 L autoclave is evacuated and rinsed with argon several times. A solution comprising 20.06 g (139 mmol) methyl-2-acetoxyacrylat (1), 0.55 mmol Rh-catalyst and 360 mL propylene carbonate are injected into the autoclave. The reaction is carried out using an initial H₂-pressure of 20 bar. Following hydrogenation 120 mL of the reaction product is transferred into a glass flask that is fixed to the Spaltrohrkolonne using a rubber joint. The following starting parameters are chosen: oil bath temperature: 85 °C, shell temperature of the Spaltrohrkolonne: 70 °C, and vacuum 8 mbar. The parameters were brought to an optimum by which the oil bath temperature is 107 °C, the sump temperature is 94 °C, the temperature of the shell of the Spaltrohrkolonne is 80 °C, the temperature at the top of the Spaltrohrkolonne is 52.5 °C, and the vacuum is adjusted to 7 mbar. The ratio of reflux and sampling is adjusted to 4:1.
Hereby, distillation starts when a sump temperature of 90 °C is reached. For yield optimization the temperature of the shell of the Spaltrohrkolonne should be stepwise increased up to 90 °C.
A change of distillation fraction is necessary when the temperature at the top of the Spaltrohrkolonne is increased to 94.5 °C as this is the start of the distillation of propylene carbonate.

The advantages of the proposed process are:
- process is operable at low pressure
- propylene carbonate can be synthesized environmentally friendly, it is characterized by low toxicity and easy biodegradability
- commercially available catalysts can be chosen
- under these conditions the disturbing opening of the maleic anhydride moiety in the catalyst ligand can be prevented
- high yield of enantioselectivity
- environmentally friendly key technology
- use of propylene carbonate allows shortening of reaction times
- economically friendly key technology

## Claims

1. A process for asymmetric hydrogenation of prochiral α-acetoxy acrylic acid esters (1) for the synthesis of enantiopure lactic acid esters (2) by the use of Rh-catalyzed bisphospholane ligands in a unit using
• an autoclave, and
• a distillation process,
where
• in a first process step α-acetoxy acrylic acid esters (1) and Rh-catalyzed bisphospholane ligands are dissolved in a solvent as substrate solution,
• in a second process step the substrate solution is added to an evacuated autoclave,
• in a third process step H₂ is fed into the autoclave,
• in a fourth process step the asymmetric hydrogenation reaction is carried out under H₂-pressure,
• in a fifth process step the reaction product composed of solvent, lactic acid esters (2), remaining α-acetoxy acrylic acid esters (1) and Rh-catalyzed bisphospholane ligands is transferred into a distillation unit, is being distilled and enantiopure lactic acid esters (2) are separated from solvent, α-acetoxy acrylic acid esters (1) and Rh-catalyzed bisphospholane ligands
**characterized in that**
• propylene carbonate is used as solvent in the first process step, and
• the distillation is performed in a Spaltrohrkolonne in the fifth process step.

2. Process according to claim 1, **characterized in that** heterocyclic phosphine ligands (3 - 12) of the following types are used in the first process step:
• Me-DuPHOS (5a)
• Et-DuPHOS (5b)
• BASPHOS (8)
• Me-cat*A*Sium M (O) (10a)
• Me-cat*A*Sium M (Square) (10c)
• Me-cat*A*Sium M (Square, 4F) (10d)
• Binaphane (11)

3. Process according to claim 1 or 2, **characterized in that** [Rh(L) (1,5-cyclooctadiene)]BF₄ catalysts or [Rh(L) (norbornadiene)]BF₄ precatalyst are used in the first process step, in which (L) stands for heterocyclic phosphine ligands that will be bound to the catalyst and then pronounced as Rh-catalyzed bisphospholane ligands.

4. Process according to claim 1 to 3, **characterized in that** the Rh-catalyzed bisphospholane ligands are put into the autoclave in their solid state, in the first step before evacuation.

5. Process according to claim 1 to 4, **characterized in that** asymmetric hydrogenation in the autoclave is carried out in the fourth process step at an initial H₂-pressure of 1 - 20 bar.

6. Process according to claim 1 to 5, **characterized in that** asymmetric hydrogenation in the autoclave is carried out in the fourth process step at a temperature of 20 - 40°C.

7. Process according to claim 1 to 6, **characterized in that** the substrate solution in the first process step contains a concentration of 0.2 to 0.5 mmol/mL α-acetoxy acrylic acid esters.

8. Process according to claim 1 to 7, **characterized in that** 0.1 to 2 mol% Rh-catalyzed bisphospholane ligand is used in the asymmetric hydrogenation reaction in the fourth process step.

9. Process according to claim 1 to 8, **characterized in that** a ethyl ester group of the prochiral substrate (1) is replaced by a methyl ester group.

10. Process according to claim 1 to 9, **characterized in that** the distillation in the Spaltrohrkolonne in the fifth process step is carried out at an oil bath temperature in the range of 107 - 112 °C.

11. Process according to claim 1 to 10, **characterized in that** the distillation in the Spaltrohrkolonne in the fifth process step is carried out at a sump temperature in the range of 90 - 98 °C.

12. Process according to claim 1 to 11 **characterized in that** the shell temperature of the Spaltrohrkolonne in the fifth process step being in the range of 70 - 90 °C.

13. Process according to claim 1 to 12 **characterized in that** the distillation temperature at the top of the Spaltrohrkolonne in the fifth process step being in the range of 52 - 95 °C.

14. Process according to claim 1 to 13, **characterized in that** the distillation pressure in the fifth process step is selected from a pressure range of 6.5 - 9 mbar.

## Patentansprüche

1. Verfahren zur asymmetrischen Hydrierung von prochiralen α-Acetoxy-Akrylsäureestern (1) für die Synthese von enantiomerenreinen Milchsäureestern (2) unter Verwendung von Rh-katalysierten Bisphospholan-Liganden in einer Vorrichtung unter Einsatz
• eines Autoklaven und
• eines Destillationsverfahrens,
in dem
• in einem ersten Verfahrensschritt α-Acetoxy-Akrylsäureester (1) und Rh-katalysierte Bisphospholan-Liganden in einem Lösungsmittel zu einer Substratlösung gelöst werden,
• in einem zweiten Verfahrensschritt die Substratlösung in einen unter Vakuum stehenden Autoklaven gegeben wird,
• in einem dritten Verfahrensschritt H₂ in den Autoklaven eingebracht wird,
• in einem vierten Verfahrensschritt die asymmetrische Hydrierungsreaktion unter H₂-Druck stattfindet,
• in einem fünften Verfahrensschritt das Reaktionsprodukt aus Lösungsmittel, Milchsäureestern (2), restlichen α-Acetoxy-Akrylsäureestern (1) und Rh-katalysierten Bisphospholan-Liganden einer Destillationsvorrichtung zugeführt wird, wo es destilliert wird und enantiomerenreine Milchsäureester (2) von Lösungsmittel, α-Acetoxy-Akrylsäureestern (1) und Rh-katalysierten Bisphospholan-Liganden getrennt werden,
**dadurch gekennzeichnet, dass**
• im ersten Verfahrensschritt Propylencarbonat als Lösungsmittel verwendet wird und
• die Destillation im fünften Verfahrensschritt in einer Spaltrohrkolonne durchgeführt wird.

2. Verfahren nach Anspruch 1, **dadurch gekennzeichnet, dass** im ersten Verfahrensschritt die folgenden Typen von heterozyklischen Phosphin-Liganden (3 - 12) eingesetzt werden:
• Me-DuPHOS (5a)
• Et-DuPHOS (5b)
• BASPHOS (8)
• Me-catASium M (O) (10a)
• Me-catASium M (quadratförmig) (10c)
• Me-catASium M (quadratförmig, 4F) (10d)
• Binaphan (11).

3. Verfahren nach Anspruch 1 oder 2, **dadurch gekennzeichnet, dass** im ersten Verfahrensschritt [Rh(L) (1,5-Cyclooctadien)]BF₄ -Katalysatoren oder [Rh(L) (Norbornadien)]BF₄-Präkatalysator eingesetzt werden, wobei (L) für heterozyklische Phosphin-Liganden steht, die sich an den Katalysator binden und dann als Rh-katalysierte Bisphospholan-Liganden bezeichnet werden.

4. Verfahren nach Anspruch 1 bis 3, **dadurch gekennzeichnet, dass** die Rh-katalysierten Bisphospholan-Liganden im ersten Verfahrensschritt vor der Herstellung des Vakuums in festem Zustand in den Autoklaven eingebracht werden.

5. Verfahren nach Anspruch 1 bis 4, **dadurch gekennzeichnet, dass** die asymmetrische Hydrierung im Autoklaven im vierten Verfahrensschritt bei einem anfänglichen H₂-Druck von 1 - 20 bar durchgeführt wird.

6. Verfahren nach Anspruch 1 bis 5, **dadurch gekennzeichnet, dass** die asymmetrische Hydrierung im Autoklaven im vierten Verfahrensschritt bei einer Temperatur von 20 - 40°C durchgeführt wird.

7. Verfahren nach Anspruch 1 bis 6, **dadurch gekennzeichnet, dass** die Substratlösung im ersten Verfahrensschritt α-Acetoxy-Akrylsäureester in einer Konzentration von 0,2 bis 0,5 mmol/mL enthält.

8. Verfahren nach Anspruch 1 bis 7, **dadurch gekennzeichnet, dass** in der asymmetrischen Hydrierungsreaktion des vierten Verfahrensschrittes 0,1 bis 2 mol% Rh-katalysierter Bisphospholan-Ligand eingesetzt wird.

9. Verfahren nach Anspruch 1 bis 8, **dadurch gekennzeichnet, dass** eine Ethylestergruppe des prochiralen Substrates (1) durch eine Methylestergruppe ersetzt wird.

10. Verfahren nach Anspruch 1 bis 9, **dadurch gekennzeichnet, dass** die Destillation in der Spaltrohrkolonne im fünften Verfahrensschritt bei einer Ölbadtemperatur im Bereich von 107 - 112 °C durchgeführt wird.

11. Verfahren nach Anspruch 1 bis 10, **dadurch gekennzeichnet, dass** die Destillation in der Spaltrohrkolonne im fünften Verfahrensschritt bei einer Sumpftemperatur im Bereich von 90 - 98 °C durchgeführt wird.

12. Verfahren nach Anspruch 1 bis 11, **dadurch gekennzeichnet, dass** die Manteltemperatur der Spaltrohrkolonne im fünften Verfahrensschritt im Bereich von 70 - 90 °C liegt.

13. Verfahren nach Anspruch 1 bis 12, **dadurch gekennzeichnet, dass** die Destillationstemperatur am Kopf der Spaltrohrkolonne im fünften Verfahrensschritt im Bereich von 52 - 95 °C liegt.

14. Verfahren nach Anspruch 1 bis 13, **dadurch gekennzeichnet, dass** der Destillationsdruck im fünften Verfahrensschritt innerhalb eines Druckbereiches von 6.5 - 9 mbar ausgewählt wird.

## Revendications

1. Un process d'hydrogénation asymétrique d'esters prochiraux d'acide α-acétoxy-acryliques (1) pour une synthèse des esters énantiopurs d'acide lactique (2) avec l'utilisation de ligands de bisphospholane catalysés par rhodium dans une unité utilisant
• un autoclave et
• un processus de distillation,
sachant que
• les esters d'acide α-acétoxy-acrylique (1) et les ligands de bisphospholane catalysés par rhodium sont dissous en solvant comme solution de substrat lors d'une première phase de process,
• que la solution de substrat est ajoutée dans un autoclave sous vide lors d'une deuxième phase de process,
• que l'autoclave est alimenté en H₂ lors d'une troisième phase de process,
• que la réaction d'hydrogénation asymétrique est réalisée sous la pression de H₂ lors d'une quatrième phase de process,
• que le produit de réaction composé du solvant, des esters d'acide lactique (2), des esters d'acide α-acétoxy-acrylique résiduels (1) et des ligands de bisphospholane catalysés par rhodium est transféré dans une unité de distillation, puis distillé et que les esters énantiopurs d'acide lactique (2) sont séparés du solvant, des esters d'acide α-acétoxy-acrylique (1) et des ligands de bisphospholane catalysés par rhodium (2) lors d'une cinquième phase de process
**caractérisé en ce que**
• le carbonate de propylène est utilisé comme solvant lors de la première phase de process et que
• la distillation est réalisée dans une Spaltrohrkolonne lors de la cinquième phase du process.

2. Processus conformément à la revendication 1, **caractérisé en ce que** sont utilisés les ligands de phosphine hétérocycliques (3 - 12) des types suivants lors de la première phase du process :
• Me-DuPHOS (5a)
• Et-DuPHOS (5b)
• BASPHOS (8)
• Me-catASium M (O) (10a)
• Me-catASium M (carré) (10c)
• Me-catASium M (carré, 4F) (10d)
• Binaphane (11)

3. Processus conformément à la revendication 1 ou 2, **caractérisé en ce que** sont utilisés des catalyseurs [Rh(L) (1,5-cyclooctadiène)]BF₄ ou des précatalyseurs [Rh(L) (norbornadiène)]BF₄ lors de la première phase du process, où (L) désigne les ligands hétérocycliques de phosphine qui seront liés au catalyseur puis déclarés ligands de bisphospholane catalysés par rhodium.

4. Processus conformément aux revendications 1 à 3, **caractérisé en ce que** les ligands de bisphospholane catalysés par rhodium sont introduits dans l'autoclave à l'état solide lors de la première étape avant la mise sous vide.

5. Processus conformément aux revendications 1 à 4, **caractérisé en ce que** l'hydrogénation asymétrique dans l'autoclave est réalisée lors de la quatrième phase du process avec une pression initiale de H₂ de l'ordre de 1 - 20 bars.

6. Processus conformément aux revendications 1 à 5, **caractérisé en ce que** l'hydrogénation asymétrique dans l'autoclave est réalisée lors de la quatrième phase du process avec une température de l'ordre de 20 - 40°C.

7. Processus conformément aux revendications 1 à 6, **caractérisé en ce que** la solution de substrat dans la première phase du process contient une concentration de 0,2 à 0,5 mmol/mL d'esters d'acide α-acétoxy-acrylique.

8. Processus conformément aux revendications 1 à 7, **caractérisé en ce qu'**il est utilisé 0,1 à 2 %mole de ligands de bisphospholane catalysés au rhodium dans la réaction d'hydrogénation asymétrique lors de la quatrième phase du process.

9. Processus conformément aux revendications 1 à 8, **caractérisé en ce qu'**un groupe d'ester éthylique du substrat prochiral (1) est remplacé par un groupe d'ester méthylique.

10. Processus conformément aux revendications 1 à 9, **caractérisé en ce que** la distillation dans la Spaltrohrkolonne lors de la cinquième phase du process est réalisée avec une température du bain d'huile de l'ordre de 107 - 112 °C.

11. Processus conformément aux revendications 1 à 10, **caractérisé en ce que** la distillation dans la Spaltrohrkolonne lors de la cinquième phase du process est réalisée avec une température du puisard de l'ordre de 90 - 98 °C.

12. Processus conformément aux revendications 1 à 11, **caractérisé en ce que** la température du corps de la Spaltrohrkolonne lors de la cinquième phase du process est de l'ordre de 70 - 90 °C.

13. Processus conformément aux revendications 1 à 12**caractérisé en ce que** la température de distillation en haut de la Spaltrohrkolonne lors de la cinquième phase du process est de l'ordre de 52 - 95 °C.

14. Processus conformément aux revendications 1 à 13, **caractérisé en ce que** la pression de distillation dans la cinquième phase du process est sélectionnée sur une plage de pression comprise entre 6,5 - 9 mbars.
